# EUROPÄISCHE PATENTANMELDUNG

(11) **EP 2 987 530 A1**
(43) Veröffentlichungstag der Anmeldung: **24.02.2016**
(21) Anmeldenummer: 15168076.6
(22) Anmeldetag: 19.05.2015
(51) Int. Cl.: A61N 1/375, A61N 1/05, A61N 1/372, A61N 1/37

(54) **HERZSTIMULATOR ZUR IMPLANTATION IN EINE HERZKAMMER**

(30) Priorität: 19.08.2014 US 201462038849 P
(71) Anmelder: BIOTRONIK SE & Co. KG, 12359 Berlin (DE)
(72) Erfinder: Dörr, Thomas, 12437 Berlin (DE); Diebold, Michael, 12169 Berlin (DE); Degen, Nicolas, 8222 Behringen (CH)
(74) Vertreter: Galander, Marcus

(57) **Zusammenfassung**

Die Erfindung betrifft einen in eine Herzkammer implantierbaren Herzstimulator, der ein Gehäuse aufweist, das Fixationselemente zur passiven Fixierung des Herzstimulators in einer Herzkammer und Elektrodenpole zur Abgabe von Stimulationsimpulsen und/oder zum Erfassen von elektrischen Potentialen aufweist. Das Gehäuse enthält eine Energieversorgungseinheit, eine mit dieser verbundene Steuereinheit sowie einen mit der Steuereinheit und der Energieversorgungseinheit verbundenen Stimulationsimpulsgenerator. Auf einer Oberfläche des Gehäuses sind mehrere Elektrodenpole verteilt, und zwischen die Elektrodenpole und den Stimulationsimpulsgenerator ist eine Schaltmatrix so geschaltet, dass der Stimulationsimpulsgenerator über die Schaltmatrix je nach deren Schaltzustand mit unterschiedlichen Elektrodenpolen elektrisch zu verbinden ist.

## Beschreibung

Die Erfindung betrifft einen in eine Herzkammer implantierbaren Herzstimulator. Derartige Herzstimulatoren sind auch als Leadless Pacemaker bekannt und besitzen ein Gehäuse, das typischerweise Fixationselemente zur aktiven oder passiven Fixierung des Herzstimulators in einer Herzkammer aufweist sowie Elektrodenpole zur Abgabe von Stimulationsimpulsen und/oder zum Erfassen von elektrischen Potentialen. Außerdem weist ein derartiger Herzstimulator typischerweise eine Energieversorgungseinheit, beispielsweise eine Batterie oder einen Generator, auf, eine mit dieser verbundene Steuereinheit sowie einen mit der Steuereinheit und der Energieversorgungseinheit verbundenen Stimulationsimpulsgenerator.

Derartige Herzstimulatoren (Leadless Pacemaker), die in eine Herzkammer implantiert werden können, sind grundsätzlich bekannt. Der Vorteil eines Herzstimulators, der direkt in eine Herzkammer implantiert werden kann, ist, dass ein derartiger Herzstimulator keine Elektrodenleitungen benötigt, die von einem Stimulationsimpulsgenerator im Gehäuse des Herzstimulators zu Stimulationselektrodenpolen in der jeweiligen Herzkammer führen. Vielmehr kann ein derartiger Herzstimulator unmittelbar selbst in der Herzkammer angeordnet werden, so dass Elektrodenpole, die der Abgabe von Stimulationsimpulsen dienen, unmittelbar am Gehäuse des Herzstimulators angebracht werden können.

In eine Herzkammer implantierbare Herzstimulatoren können auf verschiedene Art und Weise in der Herzkammer verankert werden. Beispielsweise sind Herzstimulatoren mit Fixationselementen zur aktiven Fixierung des Herzstimulators bekannt. Derartige Fixationselemente müssen betätigt werden, um sie in einer jeweiligen Herzkammer zu verankern. Ein Vorteil dieser aktiven Fixierung ist, dass mit der aktiven Fixierung typischerweise eine definierte Position und Orientierung des Herzstimulators in der jeweiligen Herzkammer erzielt werden kann.

Herzstimulatoren mit Fixationselementen zur passiven Fixierung müssen nicht aktiv fixiert werden und können daher einfacher implantiert werden, haben jedoch den Nachteil, dass auch derartige Herzstimulatoren bei der Implantation so ausgerichtet werden müssen, dass ein jeweiliger Stimulationselektrodenpol, beispielsweise eine Tipp-Elektrode, am Myokard der jeweiligen Herzkammer anliegt, damit ein jeweiliger Stimulationsimpuls effektiv wirken kann, so dass die Stimulationsreizschwelle - und damit die für eine jeweilige wirksame Stimulation des Myokards erforderliche Energie - möglichst niedrig ist. Eine derartige Ausrichtung des Herzstimulators ist nicht immer ganz einfach und setzt voraus, dass die den Herzstimulator umgebenden Trabekelstrukturen des Herzens, die Fixationselemente und der Stimulationselektrodenpol zueinander in einer geeigneten geometrischen Anordnung stehen. Darüber hinaus besteht das Problem, dass sich insbesondere passiv fixierbare Herzstimulatoren nach der Implantation geringfügig bewegen können. Es kann zu sogenannten Mikrodislokationen kommen, die einen Einfluss auf die Reizschwelle haben können.

Trotz der genannten Nachteile passiv fixierbarer Herzstimulatoren besteht der Wunsch, im Ergebnis effektiv arbeitende, passiv fixierbare Herzstimulatoren zur Implantation in einer Herzkammer zu schaffen.

Erfindungsgemäß wird diesem Wunsch mit einem in eine Herzkammer implantierbaren Herzstimulator begegnet, der ein Gehäuse aufweist, das Fixationselemente zur passiven Fixierung des Herzstimulators in einer Herzkammer und Elektrodenpole zur Abgabe von Stimulationsimpulsen und/oder zum Erfassen von elektrischen Potentialen aufweist. Das Gehäuse enthält eine Energieversorgungseinheit, eine mit dieser verbundenen Steuereinheit sowie einen mit der Steuereinheit und der Energieversorgungseinheit verbundenen Stimulationsimpulsgenerator. Auf einer Oberfläche des Gehäuses sind mehrere Elektrodenpole verteilt, und zwischen die Elektrodenpole und den Stimulationsimpulsgenerator ist eine Schaltmatrix so geschaltet, dass der Stimulationsimpulsgenerator über die Schaltmatrix je nach deren Schaltzustand mit unterschiedlichen Elektrodenpolen elektrisch zu verbinden ist.

Bei einem derartigen Herzstimulator liegt derjenige Elektrodenpol, an dem sich bei einer Stimulationsimpulsabgabe das elektrische Feld konzentriert (typischerweise die Kathode) und daher als der Stimulationselektrodenpol bezeichnet wird, nicht von vornherein fest. Vielmehr kann einer von mehreren am Gehäuse des Herzstimulators angeordneten Elektrodenpolen über die Schaltmatrix so mit dem Stimulationsimpulsgenerator verbunden werden, dass einer der Elektrodenpole als Stimulationselektrodenpol dient, während beispielsweise die anderen oder einige der übrigen Elektrodenpole als jeweiliger Gegenpol dienen.

Auf diese Weise ergibt sich ein miniaturisierter Herzstimulator, der sehr einfach und zuverlässig im Trabekelwerk einer Herzkammer, beispielsweise eines rechten Ventrikels eines menschlichen Herzens, implantiert werden kann.

Vorzugsweise beträgt die Anzahl der auf dem Gehäuse des Herzstimulators angeordneten Elektrodenpole mindestens drei und vorzugsweise mindestens vier, wobei die Elektrodenpole auf der Oberfläche des Gehäuses vorzugsweise jeweils paarweise einen wenigstens annähernd gleichen Abstand voneinander haben. Vorteilhaft ist eine möglichst gleichmäßige Verteilung der Elektrodenpole über die Oberfläche des Gehäuses des Herzstimulators, so dass es bei der Implantation des Herzstimulators nicht mehr auf dessen Orientierung ankommt.

Vorzugsweise weist der Herzstimulator eine Erfassungseinheit auf, die über die Schaltmatrix mit den Elektrodenpolen verbunden ist und die ausgebildet ist, für jeden der Elektrodenpole oder Paare von Elektrodenpolen elektrische Werte zu erfassen, die eine Stimulationserfolgsprognose für einen jeweiligen Elektrodenpol oder ein jeweiliges Paar von Elektrodenpolen erlauben. Dabei ist die Erfassungseinheit mit der Steuereinheit verbunden und die Steuereinheit ausgebildet, denjenigen Elektrodenpol oder dasjenige Paar von Elektrodenpolen auszuwählen, der oder das einen größten Stimulationserfolg verspricht. Ein derartiger Herzstimulator ist in der Lage, automatisch denjenigen Elektrodenpol als Stimulationselektrodenpol auszuwählen, für den die Stimulationsreizschwelle am niedrigsten ist. So ergibt sich beispielsweise ein rechtsventrikulärer, passiv fixierbarer Leadless Pacemaker mit einer Vielzahl von Elektrodenpolen und einer automatischen Auswahl der im Ergebnis genutzten Stimulationselektrodenpole.

Die Schaltmatrix ist vorzugsweise mit der Steuereinheit verbunden und die Steuereinheit vorzugsweise ausgebildet, denjenigen Elektrodenpol oder dasjenige Paar von Elektrodenpolen, der oder das einen größten Stimulationserfolg verspricht, zumindest für die Dauer der Abgabe eines Stimulationsimpulses mit dem Stimulationsimpulsgenerator elektrisch zu verbinden. Das heißt, das die Steuereinheit nicht nur denjenigen Elektrodenpol bestimmt, für den sich eine niedrigste Reizschwelle ergibt, sondern außerdem über die Schaltmatrix dafür sorgt, dass dieser Elektrodenpol auch tatsächlich als Stimulationselektrodenpol mit dem Stimulationsimpulsgenerator verbunden ist, wenn dieser einen Stimulationsimpuls generiert und abgibt.

Insbesondere ist es bevorzugt, wenn die Steuereinheit ausgebildet ist, die Schaltmatrix zumindest für die Dauer der Abgabe eines Stimulationsimpulses in einen Schaltzustand zu versetzen, in dem derjenige Elektrodenpol, der einen größten Stimulationserfolg verspricht, mit dem Stimulationsimpulsgenerator als dem als Kathode zu nutzenden Elektrodenpol elektrisch verbunden ist. Das bedeutet, dass die Steuereinheit die Schaltmatrix so schaltet, dass einer der Elektrodenpole, der als Stimulationselektrodenpol dienen soll, als Kathode geschaltet ist.

In Bezug auf die übrigen Elektrodenpole, die nicht als Stimulationselektrodenpol dienen, ist eine Ausführungsvariante bevorzugt, bei der die Steuereinheit ausgebildet ist, die Schaltmatrix zumindest für die Dauer der Abgabe eines Stimulationsimpulses in einen Schaltzustand zu versetzen, in dem einige oder alle übrigen Elektrodenpole als Anode derart zusammengeschaltet und mit dem Stimulationsimpulsgenerator verbunden sind, dass sich für den als Kathode geschalteten Stimulationselektrodenpol eine optimale Stromdichte ergibt. Auch dies trägt zu einer möglichst effektiven Stimulation bei und ist ein Vorteil gegenüber üblichen Herzstimulatoren, die typischerweise nur einen einzigen Stimulationselektrodenpol und einen einzigen Gegenpol aufweisen. Bei derartigen herkömmlichen Herzstimulatoren ist eine automatische Optimierung der Feldverteilung nicht möglich.

Vorzugsweise ist die Steuereinheit ausgebildet, eine Stimulationserfolgsprognose vor jeder Stimulationsabgabe oder nach jeder erfolglosen Stimulationsabgabe zu erstellen. Das bedeutet, dass die Stimulationserfolgsprognose entweder Beat-to-Beat, also vor jeder Stimulationsimpulsabgabe, erfolgt oder nur dann, wenn die Abgabe eines Stimulationsimpulses keine Stimulation des Myokards bewirkt hat. Im letztgenannten Fall weist der Herzstimulator an sich bekannte Mittel zur Stimulationserfolgskontrolle (Capture Control) auf, mit denen sich ermitteln lässt, ob es nach der Abgabe eines Stimulationsimpulses zu einer sogenannten evozierten Reizantwort kommt, die sich in einer entsprechenden elektrischen Aktivität des Myokards und einer Kontraktion des Myokards und damit der Herzkammer äußert. Derartige Mittel zur Stimulationserfolgskontrolle sind grundsätzlich bekannt. Neu ist jedoch, dass diese Mittel auch eingesetzt werden können, um einen von mehreren Elektrodenpolen eines im Herzen implantierbaren Herzstimulators als geeigneten Stimulationselektrodenpol auszuwählen.

Vorzugsweise ist die Steuereinheit ausgebildet, eine Stimulationserfolgsprognose auf Basis einer gemessenen Amplitude einer R-Zacke (R-Amplitude) und/oder auf Basis einer gemessenen Impedanz und/oder auf Basis einer gemessenen Reizschwelle und/oder auf Basis einer gemessenen Reizantwort durchzuführen. Als R-Welle oder R-Zacke wird ein in einem Elektrokardiogramm erkennbarer Verlauf des elektrischen Potentials im Myokard bezeichnet, der von einer Depolarisation der Zellen des Myokards herrührt und mit einer Kontraktion des Myokards einhergeht. Eine Kontraktion des Myokards als Ergebnis einer wirksamen Stimulationsimpulsabgabe kann auch unmittelbar beispielsweise durch Impedanzmessung erfasst werden. Darüber hinaus kann die elektrische Reizantwort des Myokards auch weitergehend ausgewertet werden, beispielsweise kann der zeitliche Abstand zwischen Stimulationsimpulsabgabe und Erfassen einer induzierten Reizantwort bestimmt und für eine Stimulationserfolgsprognose herangezogen werden. Ein kürzerer zeitlicher Abstand ist dabei ein Hinweis auf eine niedrigere Reizschwelle. Dementsprechend ist derjenige Elektrodenpol der am besten geeignete Stimulationselektrodenpol, bei dem sich eine jeweils im Vergleich mit den anderen Elektrodenpolen kürzeste Zeitdauer zwischen Abgabe eines Stimulationsimpulses und Erfassen einer evozierten Reizantwort ergibt.

Entsprechend dem Vorstehenden sind elektrische Werte, die eine Stimulationserfolgsprognose für einen jeweiligen Elektrodenpol oder ein jeweiliges Paar von Elektrodenpolen erlauben, beispielsweise die Amplitude einer R-Zacke im Elektrokardiogramm (R-Amplitude), eine gemessene Impedanz zwischen zwei Elektrodenpolen, eine mittels eines an sich bekannten Reizschwellentests erfasste Reizschwelle (Elektrodenpol-spezifisch) oder ein eine evozierte Reizantwort repräsentierendes elektrisches Signal.

Das Gehäuse des Herzstimulators ist vorzugsweise wenigstens annähernd rotationssymmetrisch. Auch dies trägt dazu bei, dass es auf eine Orientierung des Gehäuses bei der Implantation nicht ankommt, so dass der Herzstimulator einfach implantiert werden kann.

Aus gleichem Grund ist es vorteilhaft, wenn die Elektrodenpole jeweils wenigstens annähernd die gleichen Abmessungen und die gleichen elektrischen Eigenschaften haben.

Vorzugsweise ist das Verhältnis von größter räumlicher Ausdehnung zu kleinster räumlicher Ausdehnung des Gehäuses kleiner als 2 und besonders bevorzugt kleiner als 1,5 oder gar 1. Im letztgenannten Fall ist das Gehäuse beispielsweise kugelförmig

Vorzugsweise beträgt das Volumen des Gehäuses zwischen 0,5 und 2 cm³ und das Gewicht des Herzstimulator ist vorzugsweise kleiner ist als 5 gr und insbesondere kleiner als 2 gr.

Die Fixationselemente können fest oder flexibel sein und sind zur passiven Fixierung des Herzstimulators im Trabekelwerk einer Herzkammer ausgelegt. Derartige Fixationselemente werden auch als Tines bezeichnet und sind grundsätzlich bekannt. Gemäß einer bevorzugten Ausführungsvariante sind die Elektrodenpole an den Fixationselementen selbst angeordnet, beispielsweise können die Fixationselemente auch die Elektrodenpole bilden. Das ist beispielsweise dann möglich, wenn die Fixationselemente als Metalldrähte ausgebildet sind und jeweils über die Schaltmatrix elektrisch mit dem Stimulationsimpulsgenerator zu verbinden sind.

Gegebenenfalls kann der Herzstimulator zusätzlich auch dauerhafte oder temporäre Fixationselemente zur aktiven Fixierung des Herzstimulators aufweisen. Dies kann beispielsweise ein Faden sein, der an einer Punktionsstelle angenäht wird und optional resorbierbar ist, so dass nur die ersten Wochen nach einer Implantation des Herzstimulators eine aktive Fixierung besteht. In dieser Zeit können sich dann die passiven Fixationselemente stabil mit dem Trabekelwerk der jeweiligen Herzkammer verbinden. Für einen solchen Faden kann das Gehäuse des Herzstimulators mit einer Fadenhalterung, z.B. einer Öse versehen sein.

Die Energieversorgungseinheit kann eine Energiequelle sein, beispielsweise eine Batterie oder aber auch eine Vorrichtung zur Energiegewinnung, also beispielsweise ein Generator. Insbesondere kann die Energieversorgungseinheit eine Nuklearbatterie enthalten.

Die Erfindung soll nun anhand eines Ausführungsbeispiels mit Bezug auf die Figuren näher erläutert werden. Von den Figuren zeigt:
- Fig. 1:: Einen in einem rechten Ventrikel eines menschlichen Herzens implantierten Herzstimulator;
- Fig. 2:: den Herzstimulator aus Fig. 1 in vergrößerter Einzeldarstellung; und
- Fig. 3:: ein schematisches Blockschaltbild eines erfindungsgemäßen Herzstimulators.

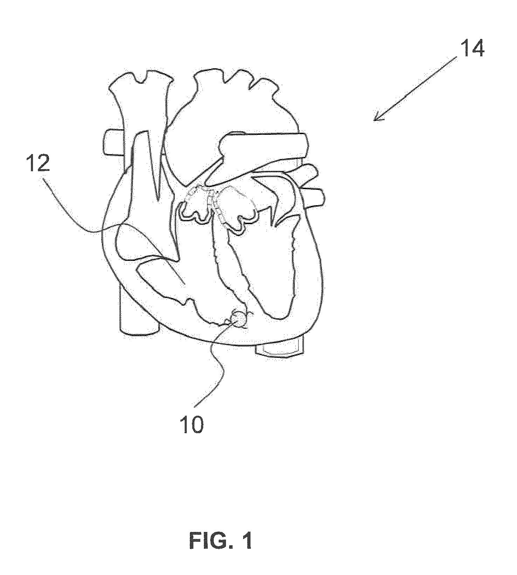

Fig. 1 zeigt einen als Leadless Pacemaker ausgebildeten Herzstimulator 10, der im Apex eines rechten Ventrikels 12 eines menschlichen Herzens 14 angeordnet ist. Dies ist der bevorzuge Implantationsort für einen derartigen Herzstimulator. Die Fixierung des Herzstimulators 10 erfolgt dabei durch "Einklemmen" und "Verhaken" des Herzstimulators 10 im - in der Figur nicht dargestellten - Trabekelwerk des Ventrikels 12. Aufgrund der nachfolgend näher beschriebenen erfindungsgemäßen Ausbildung des Herzstimulators 10 spielt die Ausrichtung des Herzstimulators 10 keine Rolle. Auf diese Weise kann erreicht werden, dass die Fixationselemente des Herzstimulators 10 ihre maximale Wirkung entfalten.

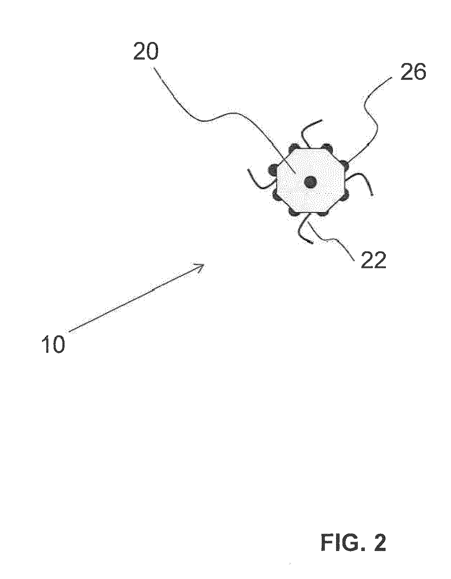

Fig. 2 zeigt den Herzstimulator 10 in einer vergrößerten Einzeldarstellung. Der Herzstimulator 10 besitzt ein Gehäuse 20, welches Fixationselemente 22 zur passiven Fixierung des Herzstimulators 10 im Trabekelwerk einer Herzkammer aufweist. Außerdem sind auf der Oberfläche des Gehäuses 20 eine Vielzahl von Elektrodenpolen 26 verteilt angeordnet.

Das Gehäuse 20 ist in der bevorzugten Ausführungsform rotationssymmetrisch ausgebildet und die Elektrodenpole 26 sind auf der Oberfläche des Gehäuses 20 im gleichen Abstand voneinander angeordnet und somit gleichmäßig verteilt. Die Elektrodenpole 26 besitzen jeweils eine identische Geometrie und damit auch identische elektrische Eigenschaften.

Die passiven Fixationselemente 22 sind als flexible Widerhaken ausgebildet und können beispielsweise von elektrisch leitenden Metalldrähten gebildet sein, so dass auch die Fixationselemente 22 alternativ oder zusätzlich als Elektrodenpole dienen können.

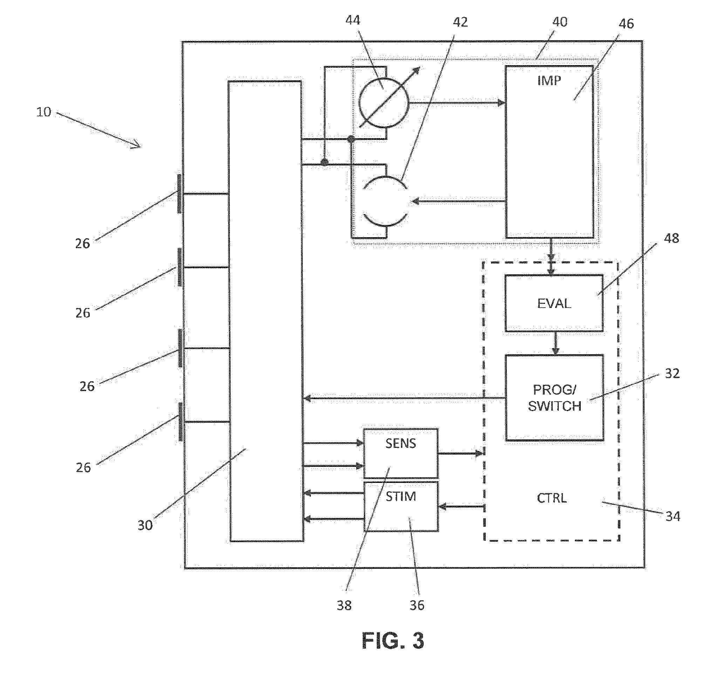

Wie eingangs erläutert besteht ein großer Vorteil des Herzstimulators 10 darin, dass einer der vielen Elektrodenpole 26 automatisch als Stimulationselektrodenpol ausgewählt werden und für die Abgabe von Stimulationsimpulsen mit einem Stimulationsimpulsgenerator verbunden werden kann. Die dazu vorzugsweise vorgesehenen Mittel sind schematisch in dem Blockschaltbild gemäß Fig. 3 dargestellt. Dieses Blockschaltbild enthält nur die für die vorliegende Erfindung relevanten Bestandteile. Weitere übliche Bestandteile eines Herzstimulators sind in dem Blockschaltbild nicht dargestellt, beispielsweise zeigt das Blockschaltbild keine Energieversorgungseinheit oder auch keine Telemetrieeinheit oder auch keinen Aktivitätssensor etc.

Schematisch dargestellt sind die Elektrodenpole 26, wobei diese in der schematischen Darstellung nicht gleichmäßig über den Umfang des Herzstimulators 10 verteilt angeordnet sind. Die Elektrodenpole 26 sind sämtlich mit einer Schaltmatrix 30 verbunden, die ihrerseits wiederum mit einer Prognose- und Schalteinheit 32 einer Steuereinheit 34 verbunden ist. Über die Prognose- und Schalteinheit 32 kann die Steuereinheit 34 des Herzstimulators 10 den Schaltzustand der Schaltmatrix 30 einstellen.

Wie Fig. 3 ebenfalls zu entnehmen ist, ist die Schaltmatrix 30 außerdem mit Aus- bzw. Eingängen eines Stimulationsimpulsgenerators 36 und einer Erfassungseinheit 38 verbunden. Der Stimulationsimpulsgenerator 36 ist ausgebildet, Stimulationsimpulse zu generieren und über die Schaltmatrix 30 und entsprechende Elektrodenpole 26 an umliegendes Gewebe abzugeben.

Die Erfassungseinheit 38 kann über die Schaltmatrix 30 mit einzelnen oder mehreren der Elektrodenpole 26 verbunden werden, so dass die Erfassungseinheit 38 über die jeweils verbundenen Elektrodenpole beispielsweise elektrische Potentialverläufe aufnehmen und erfassen und gegebenenfalls auch auswerten kann, um entsprechende Signale an die Steuereinheit 34 abzugeben.

Diese Signale kann die Steuereinheit 34 in an sich bekannter Weise auswerten, um beispielsweise bei Bedarf die Erzeugung und Abgabe eines Stimulationsimpulses auszulösen. Dazu ist die Steuereinheit 34 mit dem Stimulationsimpulsgenerator 36 verbunden.

Die Erfassungseinheit 38 kann in an sich bekannter Weise auch zur Stimulationserfolgskontrolle eingesetzt werden, so dass die Steuereinheit 34 beispielsweise einen automatischen Reizschwellentest durchführen kann, bei dem sie veranlasst, dass der Stimulationsimpulsgenerator 36 über einen jeweiligen als Stimulationselektrodenpol geschalteten Elektrodenpol und entsprechende Gegenelektroden Stimulationsimpulse unterschiedlicher Intensität abgibt, wobei nach jeder Stimulationsimpulsabgabe durch die Erfassungseinheit 38 erfasst wird, ob es zu einem Stimulationserfolg kommt. Auf diese Weise kann die Intensität bestimmt werden, die ein über einen jeweiligen Stimulationselektrodenpol abgegebene Stimulationsimpuls haben muss, um wirksam eine Stimulation des Myokards hervorzurufen. Diese Intensität ist für die jeweilige Reizwelle charakteristisch und wird typischerweise unterschiedlich sein, je nachdem welcher der Elektrodenpole 26 jeweils als Stimulationselektrodenpol mit dem Stimulationsgenerator 36 verbunden ist.

Fig. 3 zeigt außerdem noch eine Impedanzerfassungseinheit 40 mit einer Strom- oder Spannungsquelle 42, die typischerweise kurze, biphasische unterschwellige Impulse generiert, die über jeweilige Elektrodenpole an das Myokard abgegeben werden können. Eine Spannungs- oder Strommesseinheit 44 misst die mit der jeweiligen Abgabe eines Strom- oder Spannungsimpulses einhergehende Spannung bzw. Strom. Auf diese Weise ist es einer mit der Spannungs- oder Strommesseinheit 44 verbundenen Impedanzbestimmungseinheit 46 möglich, eine für die jeweilige Impedanz zwischen den Elektrodenpolen charakteristische Größe zu erzeugen und an eine Impedanzauswerteeinheit 48 in der Steuereinheit 34 abzugeben. Dies erlaubt es der Steuereinheit 34, einen Stimulationserfolg beispielsweise auch anhand von Impedanzmessungen zu erfassen, wobei der Verlauf der Impedanzwerte beispielsweise eine mechanische Kontraktion der jeweiligen Herzkammer anzeigen kann.

Abgesehen davon kann die Impedanzerfassungseinheit 40 auch dafür eingesetzt werden, mittels Impedanzmessung solche Elektrodenpole zu bestimmen, für die sich eine gute Stimulationserfolgsprognose ergibt. Damit kann die Impedanzerfassungseinheit 40 auch auf diese Weise für eine automatische Auswahl eines Stimulationselektrodenpols herangezogen werden.

Wie eingangs bereits beschrieben ist die Steuereinheit 34 dazu ausgebildet, nach Implantation des Herzstimulators 10 die hier beispielsweise beschriebenen Tests durchzuführen, um eine Stimulationserfolgsprognose für jeden der Elektrodenpole 26 zu stellen und auf diese Weise denjenigen Elektrodenpol 26 als Stimulationselektrodenpol und Kathode für die Stimulationsimpulsabgabe auszuwählen, für den sich die beste Stimulationserfolgsprognose ergibt. Da sich im Laufe der Zeit diesbezüglich Änderungen ergeben können, ist es vorteilhaft, wenn die Steuereinheit 34 die entsprechenden Tests und das Erstellen einer Stimulationserfolgsprognose nicht nur unmittelbar nach der Implantation des Herzstimulators 10 durchführt, sondern später mehrfach wiederholt.

Die Erfindung ermöglicht es, einen sehr kleinen Leadless Pacemaker zu realisieren, der auf sehr einfache und zuverlässige Weise implantiert und passiv fixiert werden kann, wobei bei der Fixierung keine Rücksicht auf die Position der Elektrodenpole genommen werden muss, weil der Herzstimulator irgendwie in das Trabekelwerk gebracht werden kann und sich dort verhakt.

### Bezugszeichenliste

- 10: Herzstimulator
- 12: Ventrikel
- 14: Menschliches Herz
- 20: Gehäuse
- 22: Fixationselemente
- 26: Elektrodenpole
- 30: Schaltmatrix
- 32: Prognose- und Schalteinheit
- 34: Steuereinheit
- 36: Stimulationsimpulsgenerator
- 38: Erfassungseinheit
- 40: Impedanzerfassungseinheit
- 42: Strom- oder Spannungsquelle
- 44: Spannungs- oder Strommesseinheit
- 46: Impedanzbestimmungseinheit
- 48: Impedanzauswerteeinheit

## Patentansprüche

1. In einer Herzkammer implantierbarer Herzstimulator (10) mit einem Gehäuse (20), das Fixationselemente (22) zur passiven Fixierung des Herzstimulators (10) in einer Herzkammer und Elektrodenpole (26) zur Abgabe von Stimulationsimpulsen und/oder zum Erfassen von elektrischen Potentialen aufweist und welches eine Energieversorgungseinheit, eine mit dieser verbundene Steuereinheit (34) sowie einen mit der Steuereinheit (34) und der Energieversorgungseinheit verbundenen Stimulationsimpulsgenerator (36) enthält, **dadurch gekennzeichnet, dass** über eine Oberfläche des Gehäuses (20) mehrere Elektrodenpole (26) verteilt sind und zwischen die Elektrodenpole (26) und den Stimulationsimpulsgenerator (36) eine Schaltmatrix (30) so geschaltet ist, dass der Stimulationsimpulsgenerator (36) über die Schaltmatrix (30) je nach deren Schaltzustand mit unterschiedlichen Elektrodenpolen (26) elektrisch zu verbinden ist.

2. Herzstimulator (10) nach Anspruch 1, **dadurch gekennzeichnet, dass** die Anzahl der Elektrodenpole (26) mindestens 3 und vorzugsweise mindestens 4 beträgt und dass die Elektrodenpole (26) auf der Oberfläche des Gehäuses (20) jeweils paarweise einen wenigstens annähernd gleichen Abstand voneinander haben.

3. Herzstimulator (10) nach Anspruch 1 oder 2, **dadurch gekennzeichnet, dass** der Herzstimulator (10) eine Erfassungseinheit aufweist, die über die Schaltmatrix (30) mit den Elektrodenpolen (26) verbunden ist und die ausgebildet ist, für jeden der Elektrodenpole (26) oder Paare von Elektrodenpolen elektrische Werte zu erfassen, die eine Stimulationserfolgsprognose für einen jeweiligen Elektrodenpol (26) oder ein jeweiliges Paar von Elektrodenpolen erlauben, wobei die Erfassungseinheit mit der Steuereinheit (34) verbunden ist und die Steuereinheit (34) ausgebildet ist, denjenigen Elektrodenpol (26) oder dasjenige Paar von Elektrodenpolen auszuwählen, der oder das einen größten Stimulationserfolg verspricht.

4. Herzstimulator (10) nach Anspruch 3, **dadurch gekennzeichnet, dass** die Schaltmatrix (30) mit der Steuereinheit (34) verbunden ist und die Steuereinheit (34) ausgebildet ist, denjenigen Elektrodenpol (26) oder dasjenige Paar von Elektrodenpolen, der oder das einen größten Stimulationserfolg verspricht, zumindest für die Dauer der Abgabe eines Stimulationsimpulses mit dem Stimulationsimpulsgenerator (36) elektrisch zu verbinden.

5. Herzstimulator (10) nach Anspruch 3 oder 4, **dadurch gekennzeichnet, dass** die Steuereinheit (34) ausgebildet ist, die Schaltmatrix (30) zumindest für die Dauer der Abgabe eines Stimulationsimpulses in einen Schaltzustand zu versetzen, in dem derjenige Elektrodenpol (26), der einen größten Stimulationserfolg verspricht, mit dem Stimulationsimpulsgenerator (36) als dem als Kathode zu nutzenden Elektrodenpol (26) elektrisch verbunden ist.

6. Herzstimulator (10) nach Anspruch 5, **dadurch gekennzeichnet, dass** die Steuereinheit (34) ausgebildet ist, die Schaltmatrix (30) zumindest für die Dauer der Abgabe eines Stimulationsimpulses in einen Schaltzustand zu versetzen, in dem einige oder alle übrigen Elektrodenpole (26) als Anode derart zusammengeschaltet und mit dem Stimulationsimpulsgenerator (36) verbunden sind, dass sich für den als Kathode dienenden Elektrodenpol (26) eine optimale Stromdichte ergibt.

7. Herzstimulator (10) nach wenigstens einem der Ansprüche 3 bis 6, **dadurch gekennzeichnet, dass** die Steuereinheit (34) ausgebildet ist, eine Stimulationserfolgsprognose vor jeder Stimulationsimpulsabgabe oder nach jeder erfolglosen Stimulationsimpulsabgabe durchzuführen.

8. Herzstimulator (10) nach wenigstens einem der Ansprüche 3 bis 7, **dadurch gekennzeichnet, dass** die Steuereinheit (34) ausgebildet ist, eine Stimulationserfolgsprognose auf Basis einer gemessenen R-Amplitude und/oder auf Basis einer gemessenen Impedanz und/oder auf Basis einer gemessenen Reizschwelle und/oder auf Basis einer gemessenen Reizantwort durchzuführen.

9. Herzstimulator (10) nach wenigstens einem der Ansprüche 1 bis 8, **dadurch gekennzeichnet, dass** das Gehäuse (20) wenigstens annähernd rotationssymmetrisch ist.

10. Herzstimulator (10) nach wenigstens einem der Ansprüche 1 bis 9, **dadurch gekennzeichnet, dass** die Elektrodenpole (26) jeweils wenigstens annähernd die gleichen Abmessungen und elektrischen Eigenschaften haben.

11. Herzstimulator (10) nach wenigstens einem der Ansprüche 1 bis 10, **dadurch gekennzeichnet, dass** die Fixationselemente (22) die Elektrodenpole (26) bilden oder beinhalten.

12. Herzstimulator (10) nach wenigstens einem der Ansprüche 1 bis 10, **dadurch gekennzeichnet, dass** das Verhältnis von größter räumlicher Ausdehnung zu kleinster räumlicher Ausdehnung des Gehäuses (20) kleiner als 2 und vorzugsweise kleiner als 1,5 ist.

13. Herzstimulator (10) nach wenigstens einem der Ansprüche 1 bis 10, **dadurch gekennzeichnet, dass** das Volumen des Gehäuses (20) zwischen 0,5 und 2 cm³ beträgt.

14. Herzstimulator (10) nach wenigstens einem der Ansprüche 1 bis 10, **dadurch gekennzeichnet, dass** dessen Gewicht kleiner ist als 5 gr.

15. Herzstimulator (10) nach wenigstens einem der Ansprüche 1 bis 10, **dadurch gekennzeichnet, dass** an dem Gehäuse (20) Mittel für eine vorübergehende aktive Fixierung, insbesondere eine Fadenhaltenmg vorgesehen sind.
